# EUROPEAN PATENT SPECIFICATION

(11) **EP 3 294 074 B1**
(45) Date of publication and mention of the grant of the patent: **22.04.2020**
(21) Application number: 16720874.3
(22) Date of filing: 09.05.2016
(51) Int. Cl.: A23L 33/16, A23C 9/152, A23C 9/18, A23L 33/125, A61K 31/7016, A61K 33/14, A23K 20/00, A23K 20/163, A23K 20/20, A23K 20/24, A23C 9/16, A61K 33/06, A23L 29/30, A23L 33/00, A23L 33/165

(54) **FAST-DISSOLVING CO-CRYSTALLINE LACTOSE**
SCHNELLLÖSLICHE CO-KRISTALLINE LAKTOSE
LACTOSE CO-CRISTALLIN À DISSOLUTION RAPIDE

(30) Priority: 11.05.2015 EP 15167141
(43) Date of publication of application: 21.03.2018
(73) Proprietor: Société des Produits Nestlé S.A., 1800 Vevey (CH)
(72) Inventor: OERTLING, Heiko, 1012 Lausanne (CH); PÄS, Malte, 3007 Bern (CH); ALZIEU, Thibaut, 1010 Lausanne (CH); DUPAS-LANGLET, Marina, 1073 Savigny (CH); ANDRIEUX, Jean-Christophe, 1066 Epalinges (CH)
(74) Representative: Couzens, Patrick John
(86) International application number: PCT/EP2016/060329
(87) International publication number: WO 2016/180776

(56) References cited:
- DE-C- 288 966
- DE-C- 305 367
- B.L Herrington: "Some Physico-Chemical Properties of Lactose:VI. The Solubility of Lactose in Salt Solutions; The Isolation of a Compound of Lactose and Calcium Chloride", Journal of Dairy Science, 31 December 1934 (1934-12-31), pages 805-814, XP055208637, DOI: 10.3168/jds.S0022-0302(34)93306-3 Retrieved from the Internet: URL:http://www.sciencedirect.com/science/a rticle/pii/S0022030234933063 [retrieved on 2015-08-19] cited in the application
- Anonymous: "Lactose - Some basic properties and characteristics", www.dfepharma.com, 28 October 2011 (2011-10-28), pages 1-12, XP055209278, http://www.dfepharma.com Retrieved from the Internet: URL:https://web.archive.org/web/2011080100 0000*/http://www.dfepharma.com/en/download s.aspx?id={2BB38A30-33EC-488D-9B1F-FEE0AD7 97636} [retrieved on 2015-08-24]
- KAZUYUKI OKU ET AL: "Interaction between Trehalose and Alkaline-Earth Metal Ions", BIOSCIENCE, BIOTECHNOLOGY, AND BIOCHEMISTRY, vol. 69, no. 1, 1 January 2005 (2005-01-01), pages 7-12, XP055208634, ISSN: 0916-8451, DOI: 10.1271/bbb.69.7
- Anonymous: "Lactose - Some basic properties and characteristics", www.dfepharma.com, 28 October 2011 (2011-10-28), pages 1-12, XP055209278, http://www.dfepharma.com Retrieved from the Internet: URL:https://web.archive.org/web/2011080100 0000*/http://www.dfepharma.com/en/download s.aspx?id={2BB38A30-33EC-488D-9B1F-FEE0AD7 97636} [retrieved on 2015-08-24]
- Pieter Walstra ET AL: "Milk Powder (Chapter 20)" In: "DAIRY SCIENCE AND TECHNOLOGY", 29 September 2005 (2005-09-29), TAYLOR & FRANCIS GROUP, BOCA RATON, XP055502542, ISBN: 978-0-8247-2763-5 vol. 10, pages 513-535,

## Description

### FIELD OF THE INVENTION

The invention relates to a powdered milk or infant formula comprising 10-50 wt% α-lactose·CaCl₂·7H₂O co-crystals and to the use of α-lactose·CaCl₂·7H₂O co-crystals for accelerating lactose dissolution.

### BACKGROUND OF THE INVENTION

The disaccharide sugar lactose, also known as milk sugar, is built from galactose and glucose linked via a β-(1,4)-glucosidic linkage. It is the most important carbohydrate of milk.

Lactose provides various nutritional benefits such as growth promotion of beneficial intestinal bacteria, for instance lactic acid bacteria (e.g. *Bifidobacterium bifidum* or *Lactobacilli*). Thereby, the consumers' resistance to intestinal infections is maintained or increased and well-being, in particular gastrointestinal health, is maintained or improved.

Moreover, the milk-derived carbohydrate lactose provides the body with energy. Due to its low glycemic index, lactose leads to a slower rise in blood sugar than other common sugars. There is consumer demand for lower glycemic index foods because of the popularity of certain diets that emphasize the reduction of glycemic load.

Due to its clean sweet taste without any aftertaste, lactose is used as carrier and stabilizer of aromas.

One shortcoming of lactose, however, is its comparably low solubility and slow dissolution. The solubility of lactose in water is limited and less compared to other commonly used sugars such as maltose, sucrose, fructose or glucose.

Due to these inherent properties, the use or applicability of lactose for the above-mentioned purposes is limited and sometimes restricted; e.g. addition of pure lactose to certain food products is not possible in the desired amounts.

However, for instance in infant formulas, addition of lactose can be beneficial to adjust the nutritional profile. Furthermore, complete dissolution of lactose and homogenization of the formulation is required such that the nutritionally recommended amount of lactose is available to the metabolism.

It is e.g. disclosed in CA 1,285,812 that for maintenance of nutritional and organoleptic qualities of the starting milk, obtaining good keeping properties and instantaneous reconstitution and dissolution in water, careful treatment, a low content of free fats, the presence of lactose generally in amorphous form and an aerated structure is required.

Thus, a need exists for lactose in readily dissolvable forms applicable in nutritional products.

Moreover, lactose is used as a carrier or stabilizer in pharmaceutical compositions, e.g. tablets etc., most of which require ingestion of water to allow adequate intake and complete dissolution. In particular for elderly, infants, people suffering from dysphagia, xerostomia or difficulty in swallowing, or people travelling, rapid and complete dissolution, preferably without the necessity of water, is desirable.

The speed of dissolution of lactose in water, milk or saliva is influenced by various parameters, for instance by temperature, pH and particle size as well as particle size distribution.

At room temperature, the solubility of lactose in a solvent, e.g. water or milk, is comparably low (vs. other mono- or disaccharides).
Thus, complete dissolution of lactose without lump formation resulting in a fully homogeneous solution, requires a considerable amount of time or, alternatively, heat or mechanical treatment, e.g. stirring or shaking.

Yet, high temperatures might result in browning reactions (Maillard reaction), which may adversely impact nutritional and organoleptic properties of a composition.

Furthermore, in contemporary automated dispensing systems utilizing individual capsules for portioned preparation of liquid food formulations, e.g. instant soups, beverages or infant formulas, preparation time is very short (commonly less than a minute) and the amount of liquid available for complete dissolution is limited. Due to the inherent machine design, additional agitation is not an option and in order to achieve a final product that is fully homogeneous and can be readily consumed, instant and complete dissolution is an absolute prerequisite to deliver a certain range of nutritional products. It has to be guaranteed that after flushing of the capsule no residual powders remain.

Accordingly, a need exists to provide readily dissolvable forms of lactose, especially in nutrition or pharmaceutics.

Another beneficial effect of lactose is the support of the stimulation of absorption and retention of important minerals such as calcium, magnesium etc.

Calcium, the most abundant mineral in the human body, is essential for bone health and teeth development and plays a role in the prevention of developing osteoporosis.

Furthermore, calcium is essential in cell physiology, in particular in its role as second messenger, i.e. an intracellular signaling mineral involved in various cellular processes such as proliferation, differentiation, migration and apoptosis. Flux of calcium ions into and out of the cytoplasm functions as a signal for various cellular processes.

Since the body does not produce minerals, it is dependent on an external supply of calcium.
An external supply of calcium may e.g. be provided by fortified nutritional products.
Fortification is an increase of the content of essential micronutrients, i.e. vitamins and minerals (e.g. calcium).
In this respect, however, selection of an appropriate form of calcium, which supplements the desired level of the mineral without affecting flavor, solubility, bioavailability, processability and organoleptic properties of the product is challenging.

Addition of calcium to milk, for example, is associated with significant difficulties.

Direct addition of calcium salts to milk is likely to result in precipitation of calcium complexes of milk proteins.

In addition, various calcium salts commonly used for fortification purposes (e.g. calcium citrate malate, tricalcium phosphate or calcium lactate) are characterized by poor flowability rendering their handling and dosage impractical. Caking can block dosing systems and result in loss of entire production batches.

Accordingly, a need exists for novel, bioavailable, solid dosing forms for the mineral calcium, which are flowable, do not aggregate and rapidly dissolve.

Thus, it is an object of the present invention to provide a fast-dissolving form of lactose and/or an efficient way of calcium fortification in nutritional or pharmaceutical compositions. The above-described drawbacks of slow lactose dissolution and calcium fortification can be overcome by nutritional or pharmaceutical compositions comprising co-crystalline lactose with calcium salts, i.e. lactose • calcium salt co-crystals, according to the present invention.

DE 288966 relates to a non-deliquescent, crystalline material resulting from the combination of lactose and calcium chloride.

In DE 305367, combinations of carbohydrates with calcium halide salts in crystalline form are disclosed.

B.L Herrington in "Some Physico-Chemical Properties of Lactose: VI. The Solubility of Lactose in Salt Solutions; The Isolation of a Compound of Lactose and Calcium Chloride", J. Dairy Science, 31 December 1934, described a compound of lactose with CaCl₂ to which the formula α-lactose·CaCl₂·7H₂O was assigned.

However, none of these documents suggests the inclusion of lactose calcium complexes or compounds into nutritional or pharmaceutical compositions or describes their dissolution kinetics.

Until present, the dissolution behavior of lactose in a nutritional or pharmaceutical composition deriving from lactose • calcium salt co-crystals has not been investigated.

### SUMMARY OF THE INVENTION

The present inventors surprisingly found that lactose provided in form of lactose • calcium salt co-crystals comprised in compositions, e.g. nutritional or pharmaceutical compositions, shows significantly improved and accelerated dissolution behavior, resulting in a homogeneous solution without lump formation.

Furthermore, it was unexpectedly found that calcium fortification of the nutritional or pharmaceutical compositions is achieved by employing calcium salts in their co-crystalline form with lactose, offering a novel crystalline, flowable and stable dosing form for supplementary mineralization.

Accordingly, in a first aspect, the present invention provides a nutritional composition comprising lactose • calcium salt co-crystals, wherein the nutritional composition is a powdered milk or infant formula and wherein the composition comprises the lactose · calcium salt co-crystals in a concentration of 10-50 wt% based on the total weight of the composition, and wherein the lactose · calcium salt co-crystals are α-lactose·CaCl₂·7H₂O.

Also disclosed is a nutritional or pharmaceutical composition selected from the group of liquids (e.g. suspensions, slurries, foams or emulsions), dry or powdery compositions, or any combination thereof, particularly a nutritional or pharmaceutical composition which is a dry or powdery composition comprising lactose • calcium salt co-crystals. Further disclosed is a nutritional or pharmaceutical composition which is a soluble powder.

The nutritional composition of the invention comprises the lactose • calcium salt co-crystals in a concentration of 10-50 wt% based on the total weight of the composition, or, more advantageously, in a concentration of 10-20 wt% based on the total weight of the composition.

Also disclosed is a nutritional or pharmaceutical composition which may comprise the lactose • calcium salt co-crystals in a concentration of 0.1-70 wt% based on the total weight of the composition, for example in a concentration of 0.1-50 wt% based on the total weight of the composition, for further example in a concentration of 1-30 wt% based on the total weight of the composition.

Such concentrations of lactose • calcium salt co-crystals provide an efficient way of formulating a soluble powdered product, that rapidly dissolves and thus delivers the required amount of lactose to the consumer, e.g. an infant, which, due to the accelerated dissolution behavior of lactose in co-crystalline form with calcium salts, is readily available.

Further disclosed is a nutritional or pharmaceutical composition which comprises the lactose • calcium salt co-crystals in a concentration of 0.01-5 wt% based on the total weight of the composition, preferably in a concentration of 0.1-3 wt% based on the total weight of the composition.

The preferable concentrations of lactose • calcium salt co-crystals allow for efficient calcium fortification of the nutritional or pharmaceutical compositions. Due to the co-crystalline form of the lactose together with the calcium salt, an efficient way of calcium dosing is provided.

Calcium fortification using lactose • calcium salt co-crystals provides a sufficient amount of calcium without affecting the flavor, solubility, bioavailability, processability, organoleptic properties and mouthfeel of the composition.

In the nutritional composition according to the invention, the lactose of the lactose • calcium salt co-crystals is α-lactose, preferably from the group of α-lactose monohydrate, anhydrous α-lactose, or combinations thereof. Anhydrous α-lactose can exist in a stable and unstable (hygroscopic) form. In a particularly preferred embodiment the lactose of the lactose • calcium salt co-crystals is α-lactose monohydrate.

Also disclosed are lactose • calcium salt co-crystals wherein the calcium salt is selected from the group of water soluble and water insoluble calcium salts. Preferably the water soluble calcium salt is selected from the group of calcium chloride, calcium bromide, calcium hydroxide, calcium acetate, calcium citrate, calcium tartrate, mono calcium phosphate, calcium lactate, calcium malate, calcium citrate malate, or hydrated forms and combinations thereof. The water insoluble calcium salt is preferably selected from the group of calcium oxide, calcium carbonate, calcium sulfate, dicalcium phosphate, tricalcium phosphate or combinations thereof.

It is expected that these co-crystals as defined above have a similar spatial arrangement in the crystalline solid state or are even structurally isomorphous and thus show similar dissolution kinetics in a liquid.

Disclosed are also lactose • calcium salt co-crystals wherein the water soluble calcium salt is selected from the group of calcium chloride, calcium bromide, calcium citrate, or combinations thereof. The calcium salt of the lactose • calcium salt co-crystals may be selected from the group consisting of calcium chloride, calcium carbonate and calcium citrate, for example it may be calcium chloride or calcium carbonate. In the invention, the calcium salt of the lactose • calcium salt co-crystals is calcium chloride.

Also disclosed is a nutritional or pharmaceutical composition comprising lactose • calcium salt co-crystals selected from the group of hydrated or non-hydrated lactose • calcium salt co-crystals, preferably hydrated lactose • calcium salt co-crystals. The lactose • calcium salt co-crystals may e.g. be hydrated when prepared (co-crystallized) from water, or non-hydrated when prepared (co-crystallized) from non-aqueous solvents, e.g. methanol, ethanol, acetone, isopropanol, glycerol, ethylacetate, n-propanol, triacetin and triethylcitrate or 1,2-propylenglycol.

In the invention, the lactose • calcium salt co-crystals of the nutritional composition are hydrated α-lactose·CaCl₂·7H₂O co-crystals.

Disclosed is a nutritional composition selected from the group of a food product, a functional food product, a dairy product, a culinary product, a confectionery product, an instant food product for providing a beverage, a nutritional supplement, or a pet food product. In the invention, the nutritional composition is a powdered milk or an infant formula.

In particular, nutritional products in the present context may be e.g. coffee creamer, instant beverage powder, instant soups, protein fortified beverages, confectionery products such as chocolate or ice cream etc.

In the sense of the present invention, infant formulas can for instance be milk- or soy-based powders.

In a further preferred embodiment of the present invention, the nutritional composition, which is a powdered milk or more preferably an infant formula, may further comprise a nutrient selected from the group consisting of fat, protein, vitamin, polyphenol, mineral or carbohydrate.

Also disclosed is a pharmaceutical composition as described above, further comprising pharmaceutically active ingredients and their salts, preferably a pharmaceutically active ingredient.

The composition is administrable enterally or parenterally. Preferably, the pharmaceutical composition is orally or rectally administrable, more preferably, the orally administrable pharmaceutical composition is administrable as a tablet, a capsule, a gel capsule, a comprimate, a hard or soft candy, a chewing gum or a pill. Particularly preferred, the tablet is a buccal, sub-lingual, or orally-disintegrating tablet. In a second preferred embodiment, the composition is administrable via a dry-powder inhaler or a nebulizer.

In a second aspect the invention relates to the use of lactose • calcium salt co-crystals for accelerating lactose dissolution, wherein the lactose • calcium salt co-crystals are α-lactose·CaCl₂·7H₂O.

Carriers may further include starches, modified starches, milk powders, carbohydrates, sugars, proteins, amino acids, fats, sweeteners, emulsifiers etc.

Disclosed are lactose • calcium salt co-crystals which are used for the preparation of a nutritional composition, which may be a food product, a functional food product, a dairy product, a culinary product, a confectionery product, a beverage, a nutritional supplement, or a pet food product. In the invention, the nutritional composition is an infant formula or a powdered milk.

Disclosed is the use of lactose • calcium salt co-crystals for preparing a powdered nutritional composition, for example a powdered infant formula or a powdered milk. Also disclosed is the use of lactose • calcium salt co-crystals for preparing a powdered nutritional composition having improved resistance to caking, for example a powdered nutritional composition having improved resistance to caking compared to an equivalent composition where the lactose and calcium salt components of the co-crystals have been replaced by a non-cocrystalline mixture of lactose and calcium salt.

Disclosed is also a process for preparing lactose • calcium salt co-crystals comprising the steps of preparing a solution comprising a calcium salt and lactose at a temperature of 50-70 °C, preferably at a temperature of 55-65 °C, cooling the solution to 25-40 °C, preferably to 30-35 °C, adding a seeding crystal of lactose • calcium salt co-crystal, allowing the formation of crystals by precipitation and isolating the obtained crystals.

One process for preparing lactose • calcium salt co-crystals comprises the steps of adding lactose and calcium salt in a concentration range of 0.5:1.5 parts by weight to 1.5:0.5 parts by weight, preferably in a concentration range of 0.8:1.2 parts by weight to 1.2:0.8 parts by weight, more preferably in a concentration range of 1:1 parts by weight, to 1 to 2 parts of water, preferably to 1 to 1.5 parts of water, more preferably to 1.5 parts of water, at 100-300 rpm, stirring the suspension at 50-70 °C, preferably at 55-65 °C, and 100-300 rpm for 10-20 minutes, cooling the solution to 25-40 °C, preferably 30-35 °C, adding seeding crystals, stirring the solution until crystal precipitation is finished and filtering the suspension, washing of the isolated co-crystals with cold ethanol at room temperature, and drying of the co-crystals at 10-50 °C, preferably at 15-45 °C, under vacuum for 0.5-4 hours and at 10-30 °C without vacuum for 36-60 hours.

Disclosed is also a process for preparing a nutritional composition comprising the steps of preparing lactose • calcium salt co-crystals as described above and adding a nutrient selected from the group consisting of fat, protein, or carbohydrate, wherein preferably the nutritional composition is selected from the group of a food product, a functional food product, a confectionery product, a dairy product, a culinary product, an instant product for providing a beverage product, a nutritional supplement, or a pet food product, preferably, wherein the nutritional composition is an infant formula.

Also disclosed is a process for preparing a pharmaceutical composition comprising the steps of preparing lactose • calcium salt co-crystals as described above and adding a pharmaceutically active ingredient.

### BRIEF DESCRIPTION OF THE FIGURES

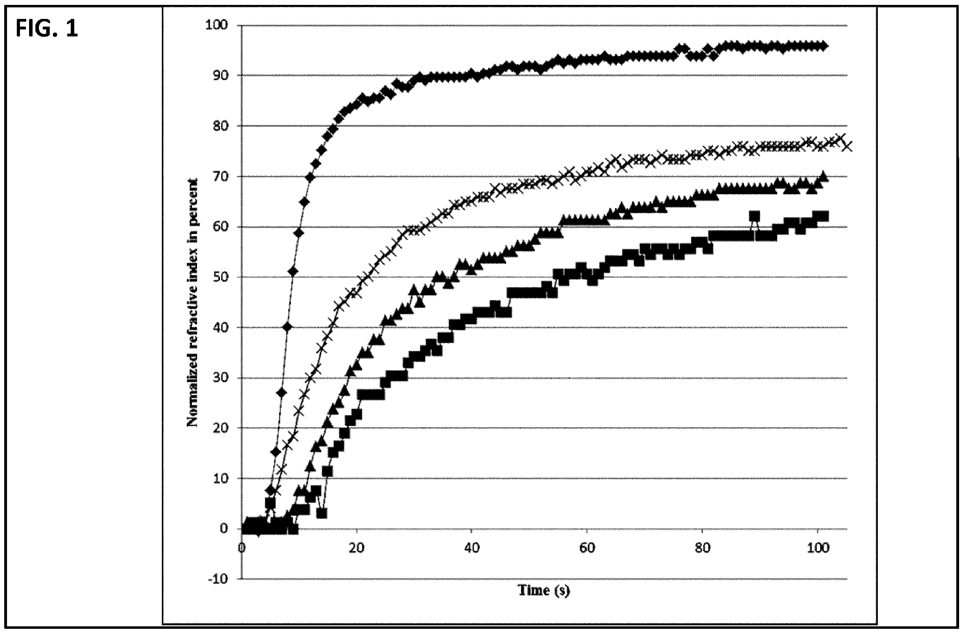

**Figure 1** displays the dissolution kinetics as the normalized refractive index in percent over time (in seconds) of lactose • CaCl₂ • 7 H₂O co-crystals in water (◆), lactose monohydrate in water (■), lactose monohydrate in a calcium chloride solution (▲) and of a physical mixture of lactose monohydrate and calcium chloride dihydrate in water (×). Dissolution kinetics were measured by online-refractometry in water over a time period of 0 to 100 seconds while stirring at 500 rpm at ambient temperature (20°C). Measurement volume of the respective solutions was 60 ml and particle size of the respective solids was comparable ranging from 60 to 90 µm. It is demonstrated that within 20 seconds about 85 % of the co-crystalline material was dissolved in water, whereas at the same time a significantly lower amount, i.e. from about 20 % to about 50 % of the lactose monohydrate or the physical mixture of lactose and calcium chloride, respectively, were dissolved.

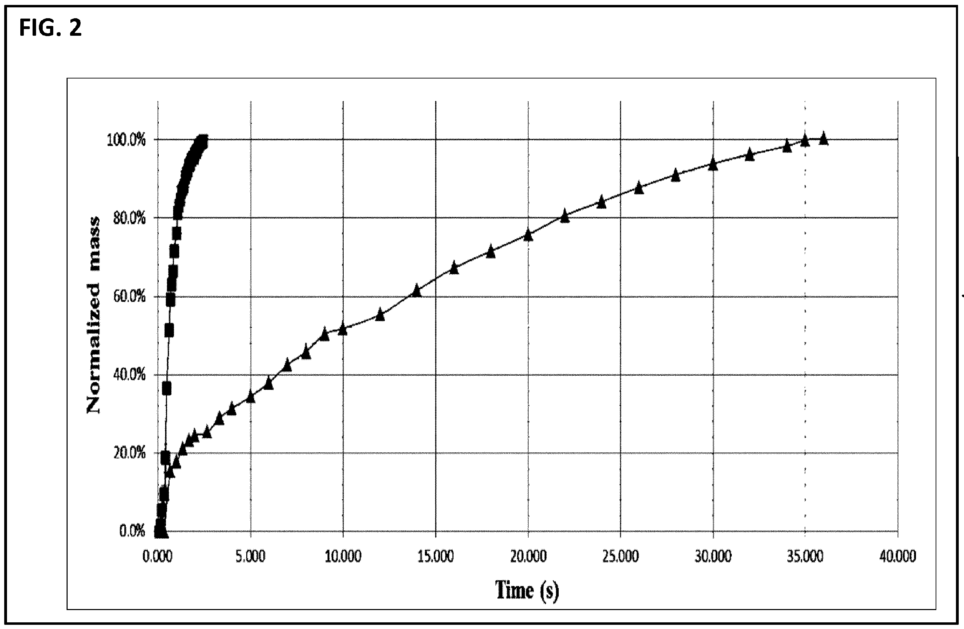

**Figure 2** shows the dissolution kinetics as normalized mass in percent over time (in seconds) for an individual lactose • CaCl₂ • 7 H₂O co-crystal (■) in comparison to an individual crystal specimen of lactose monohydrate (▲) at room temperature. The dissolution of the individual crystals was filmed under a microscope using a high-resolution camera and film-stills of the progressing dissolution were analyzed (area of remaining solid material) until complete dissolution was achieved. The liquid phase was not agitated, the selected crystals were comparable in size and morphology and the measurements were repeated multiple times.
   Dissolution kinetics were measured over a time period of 0 to 36 seconds.
   It is shown that after about 2.5 seconds, the lactose • CaCl₂ • 7 H₂O co-crystal was completely dissolved, whereas for complete dissolution of lactose monohydrate a significantly longer amount of time was necessary (that is roughly seven times as long).

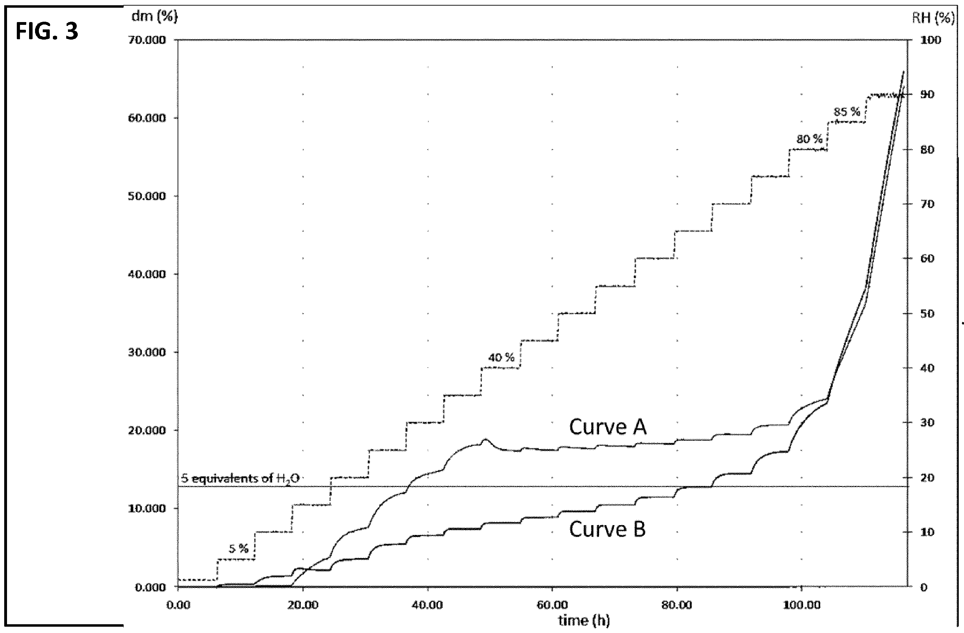

In **Figure 3** it is demonstrated that at 25 °C lactose • calcium salt co-crystals (curve B, lower curve) display an improved moisture tolerance as compared to a physical mixture of lactose monohydrate and calcium chloride dihydrate (curve A, upper curve), when measured over 120 hours. The physical mixture contains the same molar quantities of lactose and calcium chloride as compared to the co-crystalline material. The straight (horizontal) line corresponds to an uptake of five equivalents of water. dm: mass gain in percent; RH: relative humidity in percent.

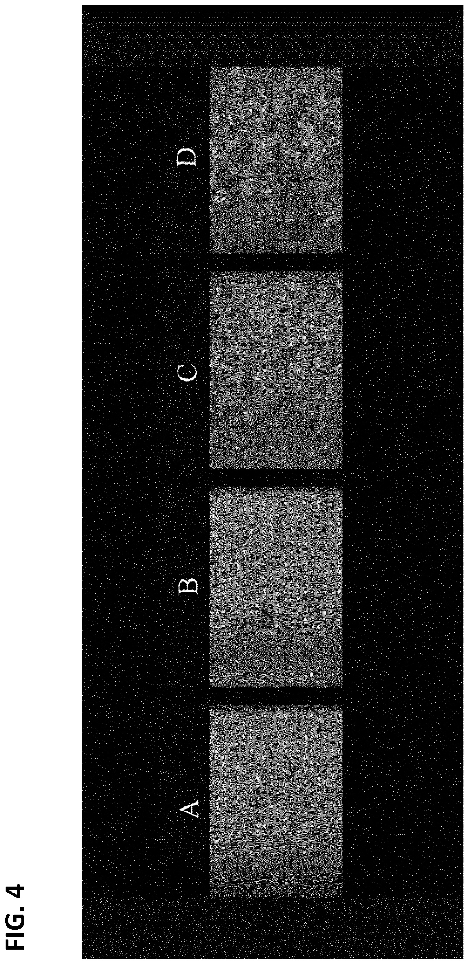

**Figure 4** shows samples of infant milk powders after storage as described in Example 7. A: no added calcium, B: with lactose · CaCl₂ · 7H₂O, C: with CaCl₂ · 2H₂O and D: with CaCl₂ · 2H₂O + lactose monohydrate.

### DETAILED DESCRIPTION OF THE INVENTION

The present invention provides a powdered milk or infant formula comprising 10-50 wt% α-lactose·CaCl₂·7H₂O co-crystals.

"Crystal" or "crystalline material" as used herein is to be understood as a solid material whose constituents are arranged in a regularly ordered pattern that is periodic in three dimensions.

"Co-crystal" according to the present invention is a crystalline structure comprising at least two components in a defined stoichiometric ratio. For instance the components are atoms, ions or molecules.

"Lactose • calcium salt co-crystals" as used herein are to be understood as lactose present in co-crystalline form with calcium salt, i.e. the crystalline structure comprises lactose and a calcium salt.

"Dissolution" as used herein means the process by which a solute forms a homogeneous solution in a solvent, e.g. water, milk, coffee, tea, juice or saliva.

"Dissolution kinetics" in the sense of the invention is defined as the rate of the physicochemical process of dissolution, i.e. the speed of dissolution.

"Fortification" as used herein is to be understood as enrichment of compositions with micronutrients such as trace elements, minerals, polyphenols, fibres and vitamins.

"Calcium fortification" means enrichment of compositions with calcium salts.

### Nutritional or pharmaceutical composition

In the present context, a "nutritional composition" may be any kind of product that provides a nutritional benefit to an individual and that may be safely consumed by a human or an animal. It may be a solid (e.g. powdery) product to be reconstituted with liquid (e.g. water, milk, coffee, tea etc.). It may be in solid, semi-solid or liquid form and may comprise one or more macronutrients, micronutrients, dietary fibers, food additives, water, etc., e.g. a protein source, a fat source, a carbohydrate source, vitamins, polyphenols and minerals. The nutritional composition may also contain antioxidants, preservatives, flavorings, coloring agents, stabilizers or emulsifiers.

A "pharmaceutical composition" as used herein is to be understood as encompassing any pharmaceutically active substance and their salts or/and a pharmaceutical carrier (excipient). The term may include any enteral or parenteral formulations such as tablets, capsules, oral liquids and injectibles. In addition to the active substance a pharmaceutical formulation may contain solubilizers, stabilizers, buffers, tonicity modifiers, bulking agents, viscosity enhancers/reducers, surfactants, chelating agents, and adjuvants.

An "active ingredient" is any substance or combination of substances that can cause a therapeutical or prophylactic effect with regard to a disorder or disease.

Advantageously, the nutritional or pharmaceutical composition is present in dry or powdery form, preferably as a powder soluble in a liquid (e.g. water, milk or saliva). Upon admixture of solvent, e.g. water, milk, etc., said composition readily dissolves until complete dissolution, i.e. a homogeneous, non-lumping solution.

Preferably, the nutritional or pharmaceutical compositions comprise a desired amount of lactose • calcium salt co-crystals to provide the consumer with a sufficient amount of lactose. Thus, the nutritional or pharmaceutical compositions comprise lactose • calcium salt co-crystals in a concentration of 0.01-100 wt% based on the total weight of the composition, preferably in a concentration of 1-70 wt% based on the total weight of the composition, more preferably in a concentration of 5-60 wt% based on the total weight of the composition. In the invention, the nutritional composition comprises lactose • calcium salt co-crystals in a concentration of 10-50 wt% based on the total weight of the composition, more preferably in a concentration of 10-20 wt% based on the total weight of the composition.

Disclosed are also lactose • calcium salt co-crystals which are present in nutritional or pharmaceutical compositions in a concentration of 0.01-5 wt% based on the total weight of the composition, preferably in a concentration of 0.1-3 wt% based on the total weight of the composition, more preferably in a concentration of 1-2 wt% based on the total weight of the composition.

Lactose • calcium salt co-crystals provide a readily dissolvable form of lactose. In comparison to pure lactose, lactose • calcium salt co-crystals dissolve considerably faster in a solvent, e.g. milk, saliva or water, giving a homogeneous, lump-free solution. Thus, the availability of lactose to the consumer's metabolism is significantly increased bringing about several advantages, for instance nutritional benefits and growth promotion of beneficial intestinal bacteria.

The dissolution kinetics of lactose • calcium salt co-crystals in nutritional or pharmaceutical compositions are improved, i.e. a significantly shorter amount of time is required for complete dissolution.

To obtain complete dissolution of pure lactose in the desired timeframe, heating, i.e. to a temperature in the range of 25-100 °C might alternatively be applied. This, however, is not necessary for the nutritional or pharmaceutical compositions comprising the lactose • calcium salt co-crystals according to the invention. A homogeneous, lump-free solution is rapidly obtained in a very short amount of time at room temperature - even without stirring. Nutritional or pharmaceutical compositions comprising lactose • calcium salt co-crystals have been found to dissolve rapidly in water at a range of temperatures. For example a milk powder comprising lactose • calcium salt co-crystals dissolved rapidly without lumping at 4°C, a temperature suitable for producing an instant cold drink, such as might be employed in a drink dispenser. An infant milk formulation comprising lactose • calcium salt co-crystals was found to dissolve rapidly without lumping at 40° C, a temperature typically used for preparing infant formula, e.g. with boiled water that has been left to cool.

Another advantage is that lactose • calcium salt co-crystals allow for efficient fortification of a food or a beverage with calcium.

On a technical scale, additional mineralization of a food product with calcium might raise several issues due to low flowability of current calcium salts commonly used for fortification purposes, which renders handling and dosage difficult.

Compositions comprising lactose • calcium salt co-crystals provide calcium in co-crystalline form combined with lactose. The co-crystals are characterized by good flowability, and improved processability, resulting in easier handling and dosage.

In addition, lactose is rendered available in combination with the essential mineral calcium. An important property of lactose is that it supports the stimulation of calcium absorption. Hence, a lower amount of calcium is required in nutritional or pharmaceutical compositions comprising lactose • calcium salt co-crystals compared to compositions fortified with pure calcium.

The lactose of the lactose • calcium salt co-crystals may be present in one of its isomers, i.e. α-lactose or β-lactose, either hydrated or anhydrous.

In crystalline α-lactose each molecule of lactose is associated with one molecule of water, i.e. crystalline α-lactose is present as α-lactose monohydrate.

### β-lactose is often referred to as anhydrous lactose.

The calcium salt of the lactose • calcium salt co-crystals may be selected from water-soluble and water-insoluble calcium salts.

Lactose • calcium salt co-crystals wherein the calcium salt is selected from a water-soluble calcium salt may be prepared from solution by direct crystallization, e.g. by solvent evaporation, slow cooling of the solution, addition of an anti-solvent etc. Alternatively, if the calcium salt is selected from a water-insoluble calcium salt, the co-crystals may be prepared by mechanical processes such as grinding, ball milling of a mixture etc.

The individual constituents of the respective co-crystal are mixed in the required molar ratio and treated mechanically in standard micronization equipment as for example ball mills, disc mills, planetary ball mills etc. for a certain amount of time. Optionally, a liquid can be added to allow for liquid-assisted grinding (LAG) or formation of stoichiometric solvates, e.g. hydrates or ethanolates.

Optionally, the desired co-crystals can also be produced by established and industrialized techniques such as spray-drying, freeze-drying, twin-screw extrusion, roller-compaction, compression or in certain cases straightforward mechanical mixing/blending. The co-crystals may be produced by spray-drying, for example by spray-drying a suitable solution comprising a calcium salt and lactose.

Nutritional or pharmaceutical compositions optionally comprise hydrated or non-hydrated lactose • calcium salt co-crystals, depending on their preparation process. If not co-crystallized from water, which generally results in hydrated lactose • calcium salt co-crystals, but with alcohols, non-hydrated lactose • calcium salt co-crystals can be obtained.

The lactose • calcium salt co-crystals comprised in the nutritional or pharmaceutical composition may be lactose • calcium chloride • 7 H₂O co-crystals.
Lactose • calcium chloride • 7 H₂O co-crystals comprise lactose and calcium chloride, preferably in an equivalent amount.

Generally, the nutritional composition as used herein may be a food product, a functional food product, a dairy product, a culinary product, a confectionery product, an instant food product for providing a beverage, a nutritional supplement, or a pet food product. In the invention, the nutritional composition is a powdered milk, more preferably, the nutritional composition is an infant formula.

"Food product" in the present context means a substance that serves as food or can be prepared as food, i.e. a substance that can be metabolized by an individual resulting in energy and/or tissue.

The term "functional food product" means a food product providing an additional health-promoting or disease-preventing function to the individual. Any kind of known biologically-active compounds may be added to the food product of this disclosure in order to provide additional health benefits.

"Dairy products", as used herein, are food products produced from animals such as cows, goats, sheep, yaks, horses, camels, and other mammals. Examples of dairy products suitable in the present invention are low-fat milk (e.g. 0.1%, 0.5% or 1.5% fat), fat-free milk, milk powder, whey and whey powders, whole milk, whole milk products, butter, buttermilk, buttermilk products, yoghurts, skim milk, skim milk products, high milk-fat products, condensed milk, creme fraîche, cream, cheese, cream cheese, spreads, ice cream, frozen yoghurts, frozen confectionery, coffee creamers and confectionery products, e.g. chocolate. Preferably, the dairy product is selected from a low-fat milk, a fat-free milk, a milk product, or a protein powder.

An "instant food product for providing a beverage" in the present context means instant powder beverage mixtures in individually-portioned or bulk form, e.g. coffee creamer, instant coffee mixtures, instant tea powder, milk powder or chocolate or chocolate milk powder, malted beverages etc.

A "beverage" is a nutritional product in liquid or semi-liquid form that may be safely consumed by an individual.

A "culinary product" in the present context means (in individually-portioned or bulk form) an instant soup, bouillon cube or bouillon powder, flavoring or powdered cooking aid or dehydrated ready-meal.

In the present context, a "nutritional supplement" describes a nutritional composition which is provided in addition to a regular diet providing nutrients (macronutrients or micronutrients) or dietary fibers, e.g. micronutrients like certain vitamins, minerals, e.g. macronutrients like fatty acids, amino acids, carbohydrates, protein etc.

A "pet food product" is a nutritional product that is intended for consumption by pets.
A pet or companion animal is an animal selected from dogs, cats, birds, fish, rodents such as mice, rats, and guinea pigs, rabbits, etc.

"Powdered milk" is to be understood as milk in the form of a powder, i.e. a dried milk or dairy product produced by evaporating milk to dryness.

Preferably, the nutritional composition of the invention is an infant formula. An infant formula can be a starter infant formula, a follow-on formula or a preterm infant formula, a milk fortifier such as a human milk fortifier, a baby food formula, a growing-up milk, an infant cereal composition, a medical food product for clinical nutrition or a supplement.

The term "infant formula" as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulas and follow-on formulas). It is also directed to a nutritional composition intended for infants and as defined in Codex Alimentarius (Codex STAN 72-1981) and Infant Specialities (incl. Food for Special Medical Purpose). The expression "infant formula" encompasses "starter infant formula" as well as "follow-up formula" or "follow-on formula". Surprisingly, the shelf life of a composition comprising lactose • calcium salt co-crystals is significantly prolonged in comparison to compositions comprising pure calcium salts. Lactose • calcium salt co-crystals unexpectedly show an improved moisture tolerance as compared to compositions comprising pure calcium salts.

Also disclosed is a pharmaceutical composition comprising a pharmaceutically active ingredient, i.e. a substance having direct effect on the cure, mitigation, treatment or prevention of a disease, thereby restoring, enhancing or maintaining physiological functions.

The pharmaceutical composition may be administered enterally or parenterally. Enteral administration may be e.g. by mouth, by gastric or duodenal feeding tube or rectally. Parenteral administration may be selected from the group of intravenous, intra-arterial, intra-muscular, intraosseous, intracerebral, intracerebroventricular, intrahecal, subcutaneous administration.

"Orally administrable" as used herein means via the mouth. "Rectally administrable" as used herein is defined as via the rectum.

Advantageously, lactose • calcium salt co-crystals are rapidly dissolvable in the consumer's saliva, resulting in a homogeneous, lump-free solution. Due to the ameliorated mouthfeel, consumer acceptance is increased and ingestion is possible for persons suffering from swallowing difficulties, e.g. infants, children or elderly. Further, patients suffering from dysphagia or xerostomia may be treated with the fast-dissolving pharmaceutical compositions of the invention.

Use The present invention is further directed to the use of α-lactose·CaCl₂·7H₂O co-crystals for accelerating lactose dissolution.

In nutritional or pharmaceutical compositions lactose • calcium salt co-crystals are especially advantageous, since fast dissolution and high availability of lactose is desired, preferably without prior requirement of heat treatment, agitation or an extended period of time.

In addition, lactose • calcium salt co-crystals are particularly advantageous for calcium fortification of nutritional compositions, since handling and dosage are improved and facilitated. Further, lactose provided in co-crystalline form with calcium salts supports calcium absorption.

Calcium fortification may be applied to nutritional compositions such as dairy compositions. Alternatively, calcium enrichment may particularly be advantageous in juices, e.g. apple or orange juices, because they can be consumed by subjects who do not or cannot consume dairy products.

Moreover, lactose • calcium salt co-crystals are characterized by a specific volume, which renders them suitable materials as carrier, filler, bulking agent or stabilizer in nutritional or pharmaceutical compositions. E.g. lactose • calcium salt co-crystals may be used as bulking agent when other ingredients such as fat, sugars, salt or protein are reduced.

"Bulking agent" means a volume or weight increasing component.

"Carrier" as used herein is to be understood as material to which substances are incorporated to improve the delivery of specific matter. Carriers may be used in drug delivery systems to prolong actions of pharmaceuticals, decrease their metabolism or reduce their toxicity.

"Stabilizer" in the present context means a substance that maintains something, e.g. a food or beverage, in a stable or constant state, e.g. with regard to their pH or texture.

"Filler" in the present sense relates to a substance, which is added to a composition to increase weight or size or to fill space.

### Process

Lactose • calcium salt co-crystals may be obtained by conducting co-crystallization in a solution or slurry mixed from the two components lactose and calcium salt.

Alternatively, lactose • calcium salt co-crystals may be prepared by grinding, e.g. manually with mortar and pestle, a ball mill or a vibratory mill. Optionally, liquid-assisted grinding may be performed to produce lactose • calcium salt co-crystals.

Lactose • calcium salt co-crystals may preferably be prepared by cooling a molten mixture, optionally a saturated solution of the two components, i.e. lactose and a calcium salt, resulting in co-crystal formation by precipitation.

Optionally, preparation of lactose • calcium salt co-crystals by cooling of a molten mixture or saturated solution of lactose and a calcium salt may require seeding with a seeding crystal.

In the present context, "seeding" means the use of a small piece of a co-crystal, i.e. a seeding co-crystal, from which larger co-crystals of the same crystalline phase are grown. Seeding is necessary to avoid spontaneous nucleation of undesired phases and therefore allows for a controlled production process of the desired material.

The seeding crystal may be prepared by co-crystallizing lactose and a calcium salt by cooling a molten mixture or a saturated solution of lactose and a calcium salt.

Optionally the preparation of a saturated solution of lactose and calcium salt is followed by slow evaporation.

One process for preparing lactose • calcium salt seeding crystals comprises the preparation of a mixture or solution, optionally a saturated solution for example a super-saturated solution, comprising lactose and a calcium salt at a temperature of 50-70 °C, optionally at a temperature of 55-65 °C, cooling the solution to 25-40 °C, optionally to 30-35 °C, until crystal precipitation occurs. Precipitated co-crystals can then be isolated, washed, e.g. with cold (8-10 °C) ethanol, and dried. Drying of the lactose • calcium salt co-crystal may be carried out at 10-50 °C, preferably 15-45 °C under vacuum for 0.5-4 hours, preferably 1-3 hours and at 10°-30 °C, preferably 15-25 °C for 36-60 hours without vacuum. The obtained lactose • calcium salt co-crystals may be used as seeding crystals, after their phase purity has been checked by appropriate methods, e.g. X-ray diffraction analysis.

In particular, lactose • calcium salt co-crystals may be prepared by a process comprising adding the two components, i.e. lactose and a calcium salt in a concentration range of 0.5:1.5 parts by weight to 1.5:0.5 parts by weight, optionally in a concentration range of 0.8:1.2 parts by weight to 1.2:0.8 parts by weight, optionally in a concentration range of 1:1 parts by weight, to 1 to 2 parts of water, optionally to 1 to 1.8 parts of water, optionally to 1.5 part of water at 100-300 rpm.

Seeding crystals obtained by a co-crystallization process may subsequently be used for preparing larger amounts of pure lactose* calcium salt co-crystals.

Lactose • calcium salt co-crystals may be prepared by cooling a saturated solution of the two components, i.e. lactose and calcium salt, using a seeding crystal and allowing precipitation of co-crystals. The seeding crystals may be introduced as a powder, or as a suspension in a liquid carrier, for example a suspension in glycerol.

The process for preparing lactose • calcium salt co-crystals may comprise the steps of preparing a solution (for example a saturated or supersaturated solution) comprising a calcium salt and lactose at a temperature of 50-70 °C, cooling the solution to 25-40 °C, adding a lactose • calcium salt co-crystal as a seeding crystal and allowing co-crystal formation. Lactose • calcium salt co-crystals may be isolated, optionally by filtration or centrifugation.

Preferably the process for preparing lactose • calcium salt co-crystals comprises the steps of preparing a solution, optionally a saturated solution, comprising a calcium salt and lactose at a temperature of 55-65 °C, cooling the solution to 30-35 °C, adding a lactose • calcium salt co-crystal as a seeding crystal and allowing co-crystal formation by precipitation. Lactose • calcium salt co-crystals may be isolated, optionally by filtration or centrifugation.

Preferably the process for preparing lactose • calcium salt co-crystals comprises the steps of preparing a solution, optionally a saturated solution, comprising a calcium salt and lactose, ensuring complete dissolution of all solids (for example at a temperature of 50-70 °C), controlling the temperature of the solution (for example by cooling) to 25-40 °C, adding a seeding crystal and allowing the formation of crystal (for example with precipitation), isolating the obtained crystals (for example by filtration or centrifugation), wherein the only seeding crystals added during the process consist of lactose•calcium salt co-crystals. The seeding crystals may be pure, for example they may be at least 95 wt.% lactose•calcium salt co-crystals, for example they may be at least 99 wt.% lactose•calcium salt co-crystals, for further example they may be at least 99.9 wt.% lactose•calcium salt co-crystals.

It is particularly preferred that the process for lactose • calcium salt co-crystal preparation comprises the steps of adding lactose and calcium salt in a concentration range of 0.5:1.5 parts by weight to 1.5:0.5 parts by weight, preferably in a concentration range of 0.8:1.2 parts by weight to 1.2:0.8 parts by weight, more preferably in a concentration range of 1:1 parts by weight, to 1 to 2 parts of water, preferably to 1 to 1.5 parts of water, optionally to 1 part of water at 100-300 rpm. The suspension is stirred at 55-65 °C and 100-300 rpm for 10-20 minutes, cooled to 30-35 °C and seeding crystals (lactose • calcium salt seeding crystals) are added. The solution may be stirred until crystal precipitation. Co-crystals may be isolated by filtering the suspension and washing the isolated co-crystals with cold ethanol (8-10 °C) at room temperature (20-25 °C). Isolated co-crystals may then be dried at 15-45 °C under vacuum for 1-3 hours and at 15-25 °C without vacuum for 36-60 hours.

Nutritional or pharmaceutical compositions comprising lactose • calcium salt co-crystals may be prepared by adding nutrients, e.g. carbohydrates, vitamins, proteins, polyphenols flavorings, or fat, or a pharmaceutically active ingredient to the lactose* calcium salt co-crystals.

### EXAMPLES

### Example 1 (not according to the invention):

Preparation of seeding crystals (lactose • calcium chloride heptahydrate co-crystals) for use in Example 2:
50.0 g of lactose monohydrate, followed by 50.0 g of calcium chloride dihydrate were added to 75.0 g of water over a period of 20 minutes at 40-65 °C at 300 rpm. The solution was cooled to 15-35 °C and stirring was continued until crystals started to precipitate.

The suspension was filtered and the isolated crystals were washed with cold (8-10 °C) ethanol at room temperature. The isolated product was dried at 15-45 °C under vacuum for 1-3 hours and at 15-25 °C without vacuum for 36-60 hours.

### Results:

Co-crystalline lactose • calcium chloride • 7 H₂O was obtained as white powder.

### Example 2 (not according to the invention) :

Preparation of lactose•calcium chloride co-crystals using seeding crystals:
100.0 g of calcium chloride dihydrate and 100.0 g of lactose monohydrate were added stepwise to 166.0 g of water. The dissolution is exothermic, so the solution heated up to a temperature of around 60 °C. The solution was stirred until all solids had dissolved. The solution was cooled to 30-35 °C. 10.0 mg of seeding crystals obtained by the process according to example 1 were added to the solution. After crystal precipitation, the suspension was filtered and the isolated crystals were washed with cold ethanol (8-10 °C) at room temperature. The isolated product was dried at 15-45 °C under vacuum for 1-3 hours and for 36-60 hours at 15-25 °C without vacuum.

### Results:

Co-crystalline lactose • calcium chloride • 7 H₂O was obtained as white powder.

### Example 3:

The dissolution kinetics of 2 g of lactose • CaCl₂ • 7 H₂O co-crystals in water (◆) in comparison to 1.24 g of pure lactose monohydrate in water (■), 1.24 g of lactose monohydrate in 0.52 g calcium chloride solution (▲) and a physical mixture of 1.24 g of lactose monohydrate and 0.52 g of calcium chloride dihydrate in water (×) were measured by refractometry at room temperature over a time period of 0 to 100 seconds while stirring the solution. Particle size of the respective solids (60-90 µm) was comparable.

### Results:

Figure 1 demonstrates that within 20 seconds about 85 % of the co-crystalline material, i.e. of the lactose • CaCl₂ • 7 H₂O co-crystals are dissolved in water, whereas at the same time only about 20 % of the lactose monohydrate was dissolved in water. Similarly, a significantly lower amount of the lactose monohydrate as compared to the co-crystalline material was dissolved in the calcium chloride solution, namely about 30 %. Of the physical mixture of lactose and calcium chloride about 50 % of the solids were dissolved in water after 20 seconds, which is a significantly lower amount than the dissolved lactose • CaCl₂ • 7 H₂O co-crystal.

### Example 4:

The dissolution kinetics of an individual α-lactose • CaCl₂ • 7 H₂O co-crystal (■) in comparison to α-lactose monohydrate (▲) in water were determined. Dissolution kinetics were measured over a time period of 0 to 36 seconds at room temperature without stirring.

### Results:

It is illustrated in figure 2 that after about 2.5 seconds, the lactose • CaCl₂ • 7 H₂O co-crystal was completely dissolved, whereas complete dissolution of lactose monohydrate required about 36 seconds. Hence, dissolution of the lactose • CaCl₂ • 7 H₂O co-crystal is about 14 to 15 times faster than dissolution of lactose monohydrate.

### Example 5 (not according to the invention) :

Dynamic vapor sorption experiments were performed for lactose • calcium salt co-crystals in comparison to a physical mixture of lactose monohydrate and calcium chloride dihydrate. The physical mixture contains the same molar quantities of lactose and calcium chloride as compared to the co-crystalline material.
Measurements were conducted over 120 hours at a temperature of 25°C and relative humidity was augmented stepwise until the samples fully liquefied.

### Results:

As illustrated in figure 3 lactose • calcium salt co-crystals (curve B, lower curve) display an improved moisture tolerance as compared to a physical mixture of lactose monohydrate and calcium chloride dihydrate (curve A, upper curve), when measured over 120 hours at a temperature of 25°C. The co-crystalline material (curve B, lower curve) gradually continued to absorb moisture in parallel with augmentation of relative humidity (RH). In contrast, the physical mixture (curve A, upper curve) absorbed moisture only until a plateau was reached at 30 % relative humidity (RH), yet at a much faster rate compared to the co-crystalline material.
The straight (horizontal) line displayed in figure 3 corresponds to an uptake of five equivalents of water. dm: mass gain in percent; RH: relative humidity in percent.

### Example 6 (not according to the invention) :

X-ray powder diffraction measurements of conventional milk powder and conventional infant milk powder in comparison to co-crystalline lactose • calcium chloride:
Conventional milk powder samples, conventional infant milk powder samples and lactose • calcium chloride heptahydrate co-crystals were analyzed via X-ray powder diffraction to investigate the presence of lactose • calcium chloride heptahydrate co-crystals in conventional milk powder or infant milk powder compositions.

### Results:

Neither in conventional milk powder, nor in conventional infant milk powder, lactose • calcium chloride heptahydrate co-crystals were detected within the detection limit of 5-7 %.
The detection limit for various analytical X-ray diffraction set-ups was established by adding pure co-crystalline material to the respective powder matrices. Hence, none of these conventional nutritional compositions comprises the lactose • calcium salt co-crystals according to the invention.

The following protocol was used:
Measurement conditions: The samples were loaded in 0.8 mm glass capillaries and X-ray powder diffraction data was collected with copper Kα1 radiation in transmission geometry using a Stoe Stadi-P powder diffractometer equipped with a curved imaging plate detector. For each sample, two patterns were collected during 3600 seconds; each over a two-theta range of -35 to 99 degrees. The raw patterns were then added, leading to increased counting statistics and truncated in the two-theta range 3 to 80 degrees to give the final pattern.

For the pure milk and infant milk powders, longer exposure times were used. Theses samples were additionally collected on a microsource diffractometer (Agilent Supernova) to remove background due to the scattering of the capillary. An empty capillary was also measured for subsequent removal of the background.

The reference peaks used for phase quantification were the three highest peaks in the pure reference sample, e.g. two-theta corresponds to 12.68, 21.58 and 22.30.

Addition of pure Lactose • CaCl₂ • 7 H₂O to the sample matrix was used to establish the detection limit. Samples containing 5, 7, 10 and 15 percent of added pure Lactose • CaCl₂ • 7 H₂O respectively were compared to the pure reference.
Consequently, the detection limit was established to be between 5 and 7 percent. At around 5 percent the highest peak is still visible, but the second and third peak tend to disappear in the background. The detection limit will also depend on the setting used for the measurement; the setting used was the transmission mode with capillaries. This requires very little material (0.8 mm capillary, beam height 0.4 mm), so that the homogeneity of the sample was carefully monitored at the same time.

Conclusion for the commercial powdered milk sample: The sample is fully amorphous. There is no trace of Lactose • CaCl₂ • 7 H₂O.

Conclusion for the commercial infant milk sample: The sample is fully amorphous. There is no trace of Lactose • CaCl₂ • 7 H₂O.

### Example 7:

The effect of various calcium-containing ingredients on storage stability was examined in powdered infant growing-up milk (*BEBA* 3, Nestle). Four mixtures were prepared:
A: 3.4g of BEBA3
B: 3.4g of BEBA3 + 1.4g of Lactose · CaCl₂ · 7H₂O
C: 3.4g of BEBA3 + 0.4g of CaCl2 · 2H2O
D: 3.4g of BEBA3 + 0.4g of CaCl₂ · 2H₂O + 0.9g of Lactose monohydrate

The mixtures were stored for one-month at 53% of relative humidity followed by 3 months at ambient temperature. The resulting powders were photographed (Figure 4). Sample B, where the calcium is added as lactose calcium salt co-crystals shows very little caking, similar to powder A where no calcium has been added. Sample C, where the calcium is added as calcium chloride showed considerable caking, with the powder D where calcium chloride is added in a simple mixture with lactose showing the most caking. Lactose • calcium salt co-crystals can be seen to provide a source of calcium which does not lead to caking problems in powders.

## Claims

1. Nutritional composition comprising lactose • calcium salt co-crystals wherein the nutritional composition is a powdered milk or infant formula and wherein the composition comprises the lactose • calcium salt co-crystals in a concentration of 10-50 wt% based on the total weight of the composition, and wherein the lactose • calcium salt co-crystals are α-lactose • CaCl₂ • 7 H₂O.

2. Nutritional composition according to claim 1, wherein the nutritional composition further comprises a nutrient selected from the group consisting of fat, protein, vitamin, polyphenol, mineral or carbohydrate.

3. Use of lactose•calcium salt co-crystals for accelerating lactose dissolution, wherein the lactose • calcium salt co-crystals are α-lactose • CaCl₂ • 7 H₂O.

## Patentansprüche

1. Nährstoffzusammensetzung, umfassend Laktose • Calciumsalz-Co-Kristalle, wobei die Nährstoffzusammensetzung eine pulverisierte Milch- oder Säuglingsformel ist und wobei die Zusammensetzung die Laktose • Calciumsalz-Co-Kristalle in einer Konzentration von 10-50 Gew.-%, bezogen auf das Gesamtgewicht der Zusammensetzung, umfasst, und wobei die Laktose • Calciumsalz-Co-Kristalle α-Laktose • CaCl₂ • 7 H₂O sind.

2. Nährstoffzusammensetzung nach Anspruch 1, wobei die Nährstoffzusammensetzung ferner einen Nährstoff, ausgewählt aus der Gruppe bestehend aus Fett, Protein, Vitamin, Polyphenol, Mineral oder Kohlenhydrat, umfasst.

3. Verwendung von Laktose•Calciumsalz-Co-Kristallen zur Beschleunigung von Laktose-Auflösung, wobei die Laktose • Calciumsalz-Co-Kristalle α-Laktose • CaCl₂ • 7 H₂O sind.

## Revendications

1. Composition nutritionnelle comprenant des co-cristaux de lactose • sel de calcium dans laquelle la composition nutritionnelle est une préparation de lait en poudre ou pour nourrissons et dans laquelle la composition comprend les co-cristaux de lactose • sel de calcium en une concentration de 10 à 50 % en poids sur la base du poids total de la composition, et dans laquelle les co-cristaux de lactose • sel de calcium sont de l'a-lactose • CaCl₂ • 7 H₂O.

2. Composition nutritionnelle selon la revendication 1, dans laquelle la composition nutritionnelle comprend en outre un nutriment choisi dans le groupe constitué de matière grasse, protéine, vitamine, polyphénol, minéral ou glucide.

3. Utilisation de co-cristaux de lactose•sel de calcium pour accélérer la dissolution de lactose, dans laquelle les co-cristaux de lactose • sel de calcium sont de l'a-lactose • CaCl₂ • 7 H₂O.
